# EUROPEAN PATENT SPECIFICATION

(11) **EP 4 241 675 B1**
(45) Date of publication and mention of the grant of the patent: **19.11.2025**
(21) Application number: 22161740.0
(22) Date of filing: 11.03.2022
(51) Int. Cl.: A61B 5/08, A61B 5/087, A61B 5/00, A61B 5/11, A61B 5/0538

(54) **METHOD AND SYSTEM FOR SENSING A SUBJECT'S BREATHING AND USE OF THE METHOD AND SYSTEM IN TREATING OBSTRUCTIVE SLEEP APNEA**
VERFAHREN UND SYSTEM ZUM ERFASSEN DER ATMUNG EINES PROBANDEN UND VERWENDUNG DES VERFAHRENS UND SYSTEM ZUM BEHANDELN VON OBSTRUKTIVER SCHLAFAPNOE
PROCÉDÉ ET SYSTÈME DE DÉTECTION DE RESPIRATION D'UN SUJET ET UTILISATION DU PROCÉDÉ ET DU SYSTÈME DANS LE TRAITEMENT DE L'APNÉE OBSTRUCTIVE DU SOMMEIL

(43) Date of publication of application: 13.09.2023
(73) Proprietor: Nyxoah SA, 1435 Mont-St-Guibert (BE)
(72) Inventor: Kohn, Sarah, Modiin 7174661 (IL); Tsukran, Roi, Rishon Le-Zion 7575721 (IL)
(74) Representative: Patentanwälte Olbricht Buchhold Keulertz

(56) References cited:
- KR-B1- 102 278 695
- US-A- 5 825 293
- US-A1- 2013 204 314
- US-A1- 2014 371 822
- US-A1- 2015 224 307
- US-A1- 2021 316 139

## Description

The invention described herein refers to a method for sensing a subject's breathing and to a system for implementing said method.

Neural modulation, i.e. electrical stimulation of nerves, is well-known in the prior art as a reliable and effective type of medical treatment. It presents the opportunity to tackle many physiological conditions and disorders by interacting with the body's own natural neural processes. Neural modulation includes inhibition (e.g. blockage), stimulation, modification, regulation, or therapeutic alteration of activity, electrical or chemical, in the central, peripheral, or autonomic nervous system. By modulating the activity of the nervous system, several different goals may be achieved. For instance, motor neurons may be stimulated at appropriate times to cause muscle contractions. Further, sensory neurons can be blocked to relieve pain or stimulated to provide a signal to a subject (i.e. a person suffering from sleep apnea). In yet other examples, modulation of the autonomy nervous system may be used to adjust various involuntary physiological parameters, such as heart rate and blood pressure. Neural modulation may provide the opportunity to treat several diseases or physiological conditions. Various devices and techniques have been used in attempts to provide optimum stimulation of a tissue of interest.

Within the content of this disclosure, the expressions "nerve stimulation", "neural stimulation", "nerve modulation" and "neural modulation" are used synonymously unless something else is apparent from the respective context. In general, the above expressions refer to the process of generating an electric field in the vicinity of a nerve or a group of nerves in order to cause contraction of one or more muscles associated to said nerves. Likewise, the expressions "subject" and "patient" are used synonymously unless something else is apparent from the respective context. Both terms refer to a person potentially suffering from OSA.

One of the conditions to which neural modulation can be applied to is obstructive sleep apnea (OSA), a respiratory disorder characterized by recurrent episodes of partial or complete obstruction of the upper airway during sleep. One of the major causes of OSA is the inability of the tongue muscles to resist negative inspiratory pressure in the pharynx due to the sleep-related loss in muscle tone. As the tongue is pulled backwards, it obstructs the upper airway, decreasing ventilation and lowering lung and blood oxygen levels. Stimulation of the hypoglossal nerve ("Hypoglossal Nerve Stimulation" or HGNS) causes the tongue muscles to contract, thereby maintaining an open, unobstructed airway. When a person without OSA is sleeping, the pharyngeal muscles - a group of muscles that form the pharynx - relax and gradually collapse, narrowing the airway. Narrowing of the airway in turn limits the effectiveness of the sleeper's breathing, causing a rise in CO₂ levels in the blood of the sleeper. The increase in CO₂ results in the pharyngeal muscles contracting to open the airway to restore proper breathing. The larger of the pharyngeal muscles responsible for upper airway dilation is the genioglossus muscle, which is one of several different muscles in the tongue.

The genioglossus muscle is responsible for the forward movement of the tongue as well as for the stiffening of the anterior pharyngeal wall. In patients with OSA, the neuromuscular activity of the genioglossus muscle is decreased compared to normal individuals, accounting for insufficient response and contraction to open the airway as compared to a normal individual. This lack of response contributes to a partial or total airway obstruction, which significantly limits the effectiveness of the sleeper's breathing. In OSA patients, there are often several airway obstruction events during the night. Because of the obstruction, there is a gradual decrease of O₂ levels in the blood (hypoxemia). Hypoxemia leads to night time arousals, which may be registered by EEG, showing that the brain awakes from any stage of sleep to a short arousal. During the arousal, there is a conscious breath or gasp, which resolves the airway obstruction. An increase in sympathetic tone activity rate through the release of hormones such as epinephrine and noradrenaline also often occurs as a response to hypoxemia. Because of the increase in sympathetic tone, the heart enlarges in an attempt to pump more blood and increase the blood pressure and heart rate, further arousing the patient after the resolution of the apnea event, as the patient returns to sleep, the airway collapses again, leading to further arousals.

In order to quantify the efficacy of neural modulation as a therapeutic method (in particular HGNS) as well as for analytical purposes, it is important that therapy response is closely monitored. This is usually achieved via through monitoring of thoracic motion and nasal and/or oral airflow of the subject, e.g. with the use of PSG (sleep study or Polysomnography) or CPAP (Continuous Positive Airway Pressure) masks.

The above methods have several disadvantages. Solutions involving airflow are not suitable for seamless implementation in existing systems for neuro stimulation. Furthermore, using masks and the like may be perceived as annoying by the subject. As for self-assessment or visualization of airway opening or tongue protrusion, these methods can be highly subjective and are therefore subject to variating results or mistakes. Furthermore, even objective visual assessments merely provide qualitative results rather than quantitative.

Other concepts of monitoring therapeutic efficacy of neural stimulation focus on breathing detection. Here, implanted intracostal respiratory sensing leads are used to monitor the patient's breathing pattern. However, this method is invasive, since implanting an electronic system that is not located in the area of the stimulation implant requires longer or even additional surgery.

US 2015/0224307 A1 describes a method of providing a sleep apnea nerve stimulation therapy to a subject including detecting a respiratory waveform of the subject with a sensor.

US 2013/0204314 A1 describes methods and systems for determining appropriate situations to treat conditions such as sleep apnea.

US 2021/0316139 A1 describes systems and methods for treating obstructive sleep apnea using an acoustic sensor configured to detect acoustic sound generated by the heart and lungs.

US 2014/0371822 A1 describes a device for controlling operation of nerve-modulating implant unit from a location external to the body of the subject.

US 5,825,293 A1 describes an apparatus for monitoring breathing and for indicating a rate of breathing outside of a predetermined rate limit.

KR 102 278 695 B1 describes methods and systems for detection of sleep apnea.

The objective technical problem of the present disclosure is to eliminate the disadvantages of the prior art and to provide a method for sensing a subject's breathing in order to determine a degree of therapy coverage with respect to the desired time periods of treatment, to facilitate quantification of the efficacy of neural modulation therapy. Furthermore, the method should improve electrical nerve stimulation in a patient associated with OSA.

### Summary of the invention

Main features of the method according to the disclosure are specified by claim 1.

According to a first aspect of the disclosure, the objective technical problem is solved by a method for sensing a subject's breathing, the method comprising:
- measuring an acoustic signal and a motion signal caused by the subject's breathing during a session of electrical nerve stimulation of the subject's hypoglossal nerve or ansa cervicalis, wherein the motion signal comprises a movement or contraction of one or more muscles related to the subject's airway;
- assigning to the measured acoustic signal and to the measured motion signal a value that is indicative of a hit rate and of an inspiratory airflow, wherein the hit rate is a quantification of a level of stimulation synchronization with the subject's inhalation phases based on the subject's breathing pattern,
wherein the movement of the one or more muscles related to the subject's airway is determined using at least one motion sensing unit, wherein the motion sensing unit comprises at least one inertial measurement unit, **characterized** in that the inertial measurement unit comprises at least one magnetometer.

The hit rate and/or of an inspiratory airflow a value allows for determining a breathing pattern as well as for synchronization of stimulation with respect to the desired periods of the subject's inhalations and/or obstructive events.

Preferably, the acoustic signal is measured using at least one acoustic sensing unit, wherein the acoustic sensing unit may in particular comprise at least one microphone. The acoustic sensing unit or the microphone may for example be located external of the subject's body. According to a preferred embodiment, the at least one sensing unit may be configured underneath the subject's chin.

The acoustic signal may comprise any noise emitted by the subject. However, the acoustic signal may preferably be a sound of the subject's breathing or snoring. The acoustic sensing unit or the microphone may be configured to capture even a slight breathing sound of a subject while asleep. Furthermore, a proprietary signal processing algorithm may be implemented, with which an inhalation phase and an exhalation phase of the subject over time may be detected.

The motion signal may comprise any motion resulted by the subject movement and sensed by a motion sensing unit. However, the motion signal may preferably be an airway muscle or chin displacement resulted by the subject's breathing. The motion sensing unit, may comprise an inertial measurement unit (IMU) and/ or a gyroscope and may be configured to capture even a slight breathing movement of a subject while asleep. Furthermore, a proprietary signal processing algorithm may be implemented, with which an inhalation phase and an exhalation phase of the subject over time may be detected.

With the above method, it is possible to reliably measure and/or monitor a subject's breathing pattern and, based thereupon, quantify a level of stimulation synchronization with the subject's inhalation phases (so-called Hit Rate Quantification). It is further possible to optimize the timing of the stimulation trains' onset with the within the subject's breathing cycle.

According to an advantageous embodiment, the method may further comprise: generating a modulation signal, the modulation signal comprising an electrical stimulation pattern; applying the modulation signal to at least one pair of electrodes associated with an implant unit implanted inside the subject's body; adjusting at least one parameter of the electrical stimulation pattern in response to the value indicative of hit rate and inspiratory airflow, i.e. in response to the measured acoustic/motion signal. This way, monitoring of the efficacy of a stimulation session can be optimized even further, since two separate effects of the stimulation are monitored independently, allowing for a clearer picture.

With the method described above, the efficacy of nerve stimulation during treatment of patients suffering from OSA, in particular the efficacy of stimulation of the hypoglossal nerve or of the ansa cervicalis, can be monitored in an easy and accurate manner. It is understood that the hypoglossal nerve innervates the genioglossus muscle and that the ansa cervicalis innervates the infrahyoid strap muscles, which are responsible for stiffening or collapsing the upper airway of a subject. The infrahyoid muscles include the sternohyoid muscle, the sternothyroid muscle, the omohyoid muscle, and the thyrohyoid muscle. Furthermore, if needed, the system allows for continuous monitoring over a desired time period, providing important information regarding the long-term effects of nerve stimulation therapy.

Muscle contraction in the sense of this disclosure may refer to muscle displacement, and these expressions are used synonymously unless indicated otherwise. Muscle contraction may in particular refer to (but is not limited to) displacement of the subject's tongue or of the other muscles associated to the subject's airway.

Movement of one or more muscles related to the subject's airway may, in the sense of this disclosure, also comprise movement of the chin of the subject, since displacement of one or more of the muscles involved in patient's airway during breathing will also result in the chin being moved. Thus, the monitoring of the patient's breathing, e.g. based on an acoustic signal and/or on a muscle displacement, may also be determined by measurement of the subject's chin movement and of the subject's breathing sounds in the chin area.

The modulation signal comprising the electrical stimulation pattern is preferably generated by an external device, which may be configured for placement underneath the subject's chin. In particular, the modulation signal may be generated by a control unit and/or by a processor, which can both be part of the external unit. The modulation signal may further be transmitted to an implant unit using a transmitting element, which may comprise an antenna or a coil associated with the control unit.

According to another embodiment, the control unit may be part of the implant unit implanted beneath the subject's skin, either instead of or in addition to a control unit of the external device. This way, the control unit may be located directly on the muscle to be monitored, e.g. on the genioglossus muscle or on one or more of the infrahyoid muscles, in particular on the sternohyiod or the sternothyroid muscle. In other words, location of the control unit it is not essential for the underlying method of this disclosure. Either way, the control unit may preferably be in electrical commutation with the at least one motion sensing unit.

The step of applying the modulation signal to at least one pair of electrodes may preferably be implemented by the implant unit. The implant unit may be implantable in a vicinity or proximity of any muscle associated with the subject's airway, e.g. the genioglossus muscle, which is innervated by the hypoglossal nerve. The implant unit may comprise an electric circuit which receives the electrical stimulation pattern through an electrical communication between the external device and the implant unit and in turn generates an electric field via at the least one pair of electrodes associated with the implant unit.

The step of determining a movement of one or more muscles related to the subject's tongue in response to the modulation signal may preferably be implemented by a motion sensing unit. The method may further be characterized in that the at least one motion sensing unit comprises at least one inertial measurement unit (IMU) and/or at least one strain gauge. According to an exemplary embodiment, movement of one or more muscles related to the subject's tongue may also be determined by detecting movement of the chin of the subject, since movement of a subject's tongue will also result in movement of a subject's chin.

Using the presented method, the physiological effect to be achieved via stimulation therapy, i.e. increase the subject's inspiratory airflow, can be monitored with higher accuracy as with known methods.

With the method described herein, the physiological condition that is directly affected by the underlying nerve stimulation therapy, i.e. muscle contraction and consequently protrusion of the subject's tongue, can be monitored in addition to monitoring the subject's breathing pattern. This way, it is possible to obtain accurate information regarding therapy efficacy. Based on the determined degree of airway opening in response to stimulation with a defined set of stimulation parameters, it is further possible to adjust those parameters in order to improve therapy efficacy. For example, if a set group of stimulation parameters is not sufficient to cause tongue protrusion and open the airway, the underlying stimulation parameters may be adjusted accordingly, e.g. by increasing stimulation intensity or, if a set group of stimulation parameters is not sufficient to cause desired hit rate, the underlying stimulation parameters may be adjusted accordingly, e.g. by adjusting the stimulation cycle length and distribution.

According to a further embodiment of the method, the time periods indicative of subject's inhalation phases and obstruction events are detected, and subsequently the level of stimulation synchronization with these periods is quantified, e.g. based on an acoustic signal and/or on a muscle displacement.

According to one embodiment disclosed herein, the method may further comprise the step: adjusting at least one parameter of the electrical stimulation pattern in response to the hit rate and/or of an inspiratory airflow and/or in responds to the determined time periods indicative of subject's inhalation phases and upper airway obstruction events. This way, feedback-based or closed loop stimulation method is provided, wherein the efficacy of an ongoing stimulation session is determined by measuring the breathing sound of the subject or the displacement of the airway muscles during inhalation (i.e. detection of inhalation phases and upper airway obstruction events), and then the stimulation parameters are adjusted in response to the measured degree of synchronization of the stimulation with these events (i.e. estimated Hit Rate). The detection of such breathing events can for example be determined by only measuring an acoustic signal, or by detecting movement of the muscles associated with an inhalation in the airway or by detecting movement of the subject's chin in addition to measuring the acoustic signal. Adjustment of the at least one stimulation parameter can occur automatically or by hand (e.g. through a physician or through the subject themselves) or both. In case of automatic adjustment, the at least one stimulation parameter can be changed after a set time delay or more or less instantly (i.e. in real-time). This process may be automatically repeated in an iterative manner until one of the automatic adjustments of the stimulation parameters results in determination of a value that is indicative of a desired degree of synchronization of the stimulation with subject's inhalation and/or upper airway obstructive events.

In order to achieve a feedback-based or closed loop stimulation session, the control unit may be configured to perform logical operations such as comparing the determined Hit Rate - which is based on data corresponding the breathing sounds of the subject determined by the acoustic sensing unit or, in addition, based on data corresponding to the movement of one or more muscles related to the subject's inhalation determined by the motion sensing unit - to a pre-defined value and adjust one or more of the stimulation parameters accordingly. For example, in case the hit rate is determined in based on the measured acoustic signal below the pre-determined value, the control unit may be configured to adjust the stimulation train length and interval.

Advantageously, the movement of the muscles related to the subject's airway may comprise a muscle contraction, a change of the tongue's position and/or a change of the tongue's spatial orientation within the subject, and/or movement of the subject's chin. In particular but not exclusively, muscle contraction may refer to contraction of the genioglossus muscle, especially when innervated by the hypoglossal nerve. It is also possible that the movement of the one or more muscles related to the subject's tongue is determined using at least one motion sensing unit. The method may further be characterized in that the at least one motion sensing unit comprises at least one inertial measurement unit and/or at least one strain gauge. According to the present invention, the inertial measurement unit comprises at least one magnetometer.

The at least one parameter may comprise a pulse train amplitude, a pulse train length, a single pulse frequency, a single pulse duration, a hold duration, a pulse train interval, a duty cycle, a delay time, a ramp duration, a step-down amplitude, a ramp at train onset duration, and/or a confirmatory pulse/train amplitude. The stimulation parameters may be adjusted automatically based on the value assigned to the measured hit rate and degree of airway opening, or the parameters can be adjusted by a physician or by the patients themselves upon receiving information that the current stimulation parameters are not sufficient.

According to another aspect of this invention, a system for implementing the method according to any of the preceding claims, the system comprising:
- an implant unit configured for implantation in the vicinity of the any muscle associated with the subject's airway opening,
   wherein the implant unit comprises a pair of electrodes;
- an external device configured for communication with the implant unit;
- at least one acoustic sensing unit configured for measuring an acoustic signal;
- at least one motion sensing unit configured for measuring a movement of one or more muscles related to the subject's airway, the at least one motion sensing unit comprising at least one inertial measurement unit, wherein the at least one inertial measurement unit comprises a magnetometer; and
- a control unit configured to assign to the measured acoustic signal and to the measured motion signal a value that is indicative of a hit rate and of an inspiratory airflow,
   an wherein the control unit is further configured to transmit stimulation input to the implant unit,
wherein the hit rate is a quantification of a level of stimulation synchronization with the subject's inhalation phases based on the subject's breathing pattern.

The system may preferably be configured for implementing the method described herein.

According to a preferred embodiment, the motion sensing unit is a microphone that is part of the external device. This way, since the external device is placed externally on the subject in proximity to the implant unit, i.e. underneath the subject's chin, the acoustic sensing unit, being part of the external device, is automatically placed in an advantageous location for detecting an acoustic signal, e.g. breathing sounds of the subject.

In addition to the above, the system may comprise a motion sensing unit for determining movement of one or more muscles related to the subject's airway in response to the modulation signal applied to the at least one pair of electrodes.

Furthermore, the system comprises a control unit. The control unit may be part of the external device. In that case, the control unit may transmit modulation or stimulation input signals, e.g. the stimulation parameters, and power to the implant unit. The control unit may further receive the determined data corresponding to the breathing sounds of the subject determined by the acoustic sensing unit or, in addition, data corresponding to the movement of one or more muscles related to the subject's airway determined by the motion sensing unit, and assign a value to the assigned degree of airway opening. According to a preferred embodiment, the control unit is also able to adjust at least one of the stimulation parameters in response to the value indicative of the hit rate and/or inspiratory airflow.

Accordingly, the control unit may comprise one or more processors, which may include any electric circuit that configured to perform a logic operation on at least one input variable. The at least one processor of the control unit may therefore include one or more integrated circuits, microchips, microcontrollers, and microprocessors, which may be all or part of a central processing unit (CPU), a digital signal processor (DSP), a field programmable gate array (FPGA), or any other circuit known to those skilled in the art that may be suitable for executing instructions or performing logic operations.

The implant unit may be configured for implantation in a location that permits it to modulate a nerve (e.g. the hypoglossal nerve). The implant unit may be located in a subject such that intervening tissue exists between the implant unit and the nerve to be modulated. Intervening tissue may include muscle tissue, connective tissue, organ tissue, or any other type of biological tissue. Thus, location of the implant unit does not require immediate contact with the nerve for effective neuromodulation. The implant unit may also be located directly adjacent to nerve, such that no intervening tissue exists.

In treating OSA, the implant unit may be located on the genioglossus muscle of the patient or subject. Such a location is suitable for modulation of the hypoglossal nerve, branches of which run inside and innervate the genioglossus muscle. However, the implant unit may also be configured for placement in other locations.

The external device may be configured for location external to a patient, either directly contacting the skin or close to the skin of the patient. The external device may further be configured to be affixed to the patient, for example, by adhering to the skin of the patient, or through a band or other device configured to hold the external device in place. Adherence to the skin of the external unit may occur such that it is in the vicinity or in the proximity of the location of the implant unit. Preferably, the external device comprises the at least one acoustic sensing unit (e.g. the microphone)

The external device may be configured for fixation to the patient. For example, the external device may be configured for placement underneath the subject's chin and/or on the front of patient's neck. The suitability of placement locations may be determined by communication between external device and implant unit. The external device may comprise a housing, wherein the housing may be any suitable container configured for retaining electrical components. In addition, the housing may be any suitable size and/or shape and may be rigid or flexible. Examples of housings for the external device may include one or more of patches, buttons, or other receptacles having varying shapes and dimensions and constructed of any suitable material. The external device may be configured to adhere to a desired location. Accordingly, in some embodiments, at least one side of the housing may include an adhesive material. The adhesive material may include a biocompatible material and may allow for a patient to adhere the external unit to the desired location and remove the external device upon completion of use. The adhesive may be configured for single or multiple uses of the external unit. Suitable adhesive materials may include, but are not limited to biocompatible glues, starches, elastomers, thermoplastics, and emulsions.

According to one embodiment, the at least one motion sensing unit may be part of the implant unit and/or the at least one motion sensing unit may be part of the external device. It is also possible that both the implant unit and the external device comprise a motion sensing unit. If this is the case, the motion sensing units can be different form each other, i.e. the motion sensing unit of the internal unit may be an strain gauge and/or a gyroscope and the motion sensing unit of the external device may be an gyroscope. For example, if the motion sensing unit is part of the external device, the motion sensing unit may preferably be a IMU configured to detect chin movement, since the external device is preferably located underneath the subject's chin and since a stimulation event resulting in contraction of one or more muscles related the airway or to tongue movement will also result in movement of the subject's chin. If the motion sensing unit is part of the implant unit, the motion sensing unit may preferably comprise a strain gauge, either as a stand-alone component or together with an IMU, as a strain gauge is suitable for detecting and measuring muscle contraction and an IMU is suitable for detecting slight motion movements induced by the respiration phases and muscle collapse.

Thus, with the system described herein, it is possible to monitor at least one physiological effect of nerve stimulation of one or more nerves responsible for the muscles controlling a subject's airway (i.e. the subject's breathing sounds) via implementation of an acoustic sensing unit and, based on the measured acoustic signal determine a degree of airway opening. Furthermore, the system may be configured to automatically adjust at least one or more stimulation parameters to improve stimulation based on the degree of airway opening determined this way. For example, it is possible to quantify a level of stimulation synchronization with the subject's inhalation phases (Hit Rate Quantification) using the described system.

Advantageously, the degree of airway opening can be determined based on a detected movement of one or more muscles related to the subject's airway in addition to being determined based on a measured acoustic signal (e.g. breathing sound of the subject).

According to an example not covered by the present invention, a use of the method disclosed herein for treatment of obstructive sleep apnea is presented. The treatment of obstructive sleep apnea, for which the method can be used, may for example comprise the following steps:
- receiving a modulation signal at an implant unit implanted at an internal location on an underside of a subject's chin (e.g.in the vicinity of the genioglossus muscle);
- applying the modulation signal to at least one pair of electrodes associated with the implant unit to generate an electric field;
- and causing modulation of a hypoglossal nerve or of an ansa cervicalis of the subject in response to the electric field generated by the at least one pair of electrodes, wherein the modulation of the hypoglossal nerve may be confined to a medial branch of the hypoglossal nerve and may be initiated from a single modulation site along the medial branch.

Therefore, the at least one pair of modulation electrodes may be configured for implantation through derma on the underside of the subject's chin and for location proximate to terminal fibers of the medial branch of the subject's hypoglossal nerve. In addition, the implantable unit and the electrodes may be configured to cooperate in order to generate an electric field adapted to modulate one or more of the terminal fibers of the medial branch of the hypoglossal nerve.

Any one of the embodiments, examples or features disclosed herein may be used in combination or separately and in conjunction with any one of the aspect of the disclosed subject matter.

### Brief description of the drawings

The accompanying drawings, which are incorporated in and constitute a part of this specification, illustrate several examples of the disclosed subject matter. The drawings show the following:
- Fig. 1: shows a schematic depiction of the system disclosed herein according to a first embodiment;
- Fig. 2: shows a schematic depiction of a system according to a second embodiment;
- Fig. 3: shows a schematic depiction of a system according to a third embodiment

### Detailed description of the drawings

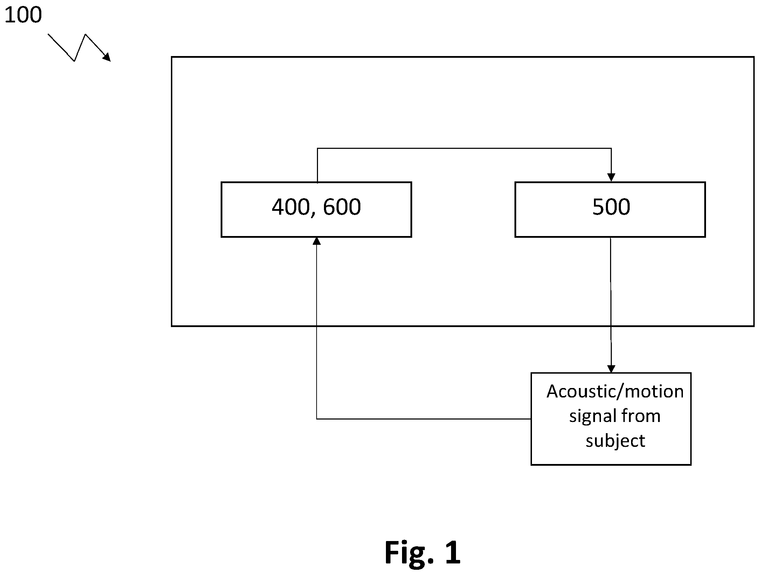

Fig. 1 shows a schematic depiction of a system 100 for measuring a degree of hit rate and/or respiratory airflow, e.g. based on an acoustic signal and/or on a muscle displacement caused by a subject breathing, e.g. in response to electrical nerve stimulation, wherein said acoustic signal may include the subject's breathing sounds and wherein said muscles may include movement of the tongue, the genioglossus muscle or one or more of the infrahyoid strap muscles. The flow diagram of Fig. 1 may serve as an example of implementation of the method. According to Fig. 1, a neuro stimulation system 100 configured for implementing the method comprises a control unit 500 and an acoustic sensing unit 400. Furthermore, the system 100 may preferably comprise an implant unit 200 (not shown) configured for implantation in the proximity of any muscle associated with the subject's airway, e.g. the genioglossus muscle, and an external device 300 (not shown) configured for electrical communication with the implant unit 200.

The implant unit 200 of the system 100 with which the method may be carried out, may be implanted in a vicinity to the genioglossus muscle, which is innervated by the hypoglossal nerve, or to one of the infrahyoid muscles, which are innervated by the ansa cervicalis. The implant unit 200 may further comprise a transmitting element, which may comprise a secondary antenna or a coil, and an electric circuit which receives an electrical stimulation pattern through the electrical communication between the external device 300 and the implant unit 200. Upon receiving the electrical stimulation pattern, the electric circuit may generate an electric field via at the at least one pair of electrodes associated with the implant unit 200. If the electric field is strong enough and/or close enough to the hypoglossal nerve, the nerve will be stimulated and innervate the genioglossus muscle, which will in turn contract. The specifications of the implant unit 200 are not limited to a certain embodiment as long as the implant unit 200 is configured for use with the method described herein.

The external device 300 of the system 100 with which the method may be carried out, may be configured for location external to a patient, in particular underneath the subject's chin and/or on the front of patient's neck. The external device 300 may therefore be configured for affixation to the patient, for example, by adhering to the skin of the patient, or through a band or other device configured to hold the external device in place. The external device 300 may preferably comprise a housing, wherein the housing can be any suitable container configured for retaining electrical components. In addition, the housing may be any suitable size and/or shape and may be rigid or flexible. Examples of housings for the external device 300 may include one or more of patches, buttons, or other receptacles having varying shapes and dimensions and constructed of any suitable material. The specifications of the external device 300 are not limited to a certain embodiment as long as the implant unit 200 is configured for use with the method described herein.

In order to measure an acoustic signal (i.e. a breathing noise sound emitted by the subject) caused by the subject, the system 100 comprises an acoustic sensing unit 400, which may be a microphone. In order to measure an motion signal caused by the subject, the system 100 comprises a motion sensing unit 600, which may be an inertial measurement unit, an accelerometer, at least one gyroscope and/or at least one magnetometer
Lastly, the system 100 according to Fig. 1 may comprise a control unit 500, for example as part of the external device 300. The control unit 500 may transmit modulation or stimulation input signals, e.g. the stimulation parameters, and power to the implant unit 200.

According to the method depicted in Fig. 1, the control unit 500 may also receive data corresponding to an acoustic signal measured by the acoustic sensing unit 400 or to a motion signal measured by the motion sensing unit, and assign to it a value relating to a hit rate and/or inspiratory airflow. According to a preferred embodiment of the method, the control unit 500 is also able to adjust at least one of the stimulation parameters in response to the value indicative of the hit rate and/or of inspiratory airflow. Accordingly, the control unit 500 may comprise one or more processors, which may include any electric circuit that configured to perform a logic operation on at least one input variable. The at least one processor of the control unit 500 may for example include one or more integrated circuits, microchips, microcontrollers, and microprocessors, which may be all or part of a central processing unit (CPU), a digital signal processor (DSP), a field programmable gate array (FPGA), or any other circuit known to those skilled in the art that may be suitable for executing instructions or performing logic operations. This way, a method for feedback-based or closed loop neuro stimulation is provided, wherein the efficacy of an ongoing stimulation session is determined by measuring the degree of airway opening and then automatically adjusting the stimulation parameters in response to the measured degree of airway opening.

In accordance with Fig. 1, the control unit 500 can be part of the external device 300, of the implant unit 200, or of both the external device 300 and the implant 200.

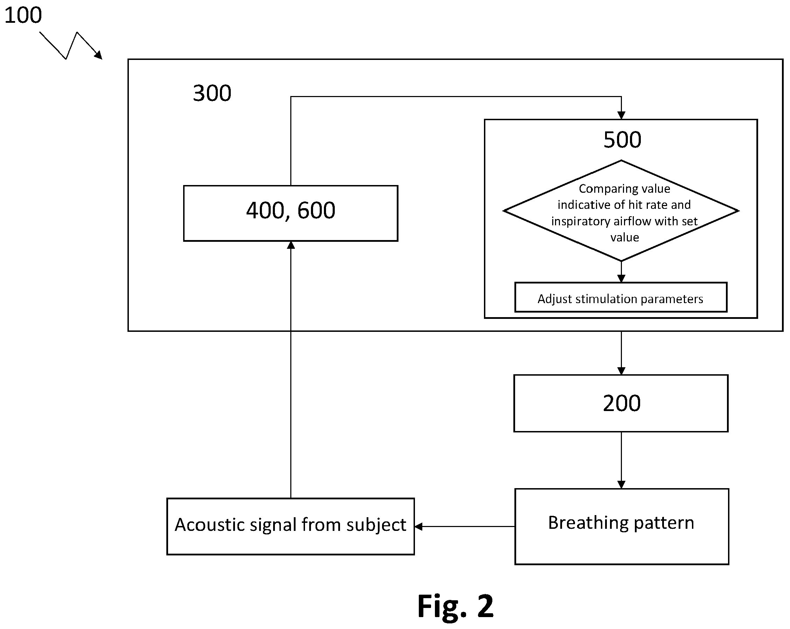

Fig. 2 shows a schematic depiction of a system 100 according to a second embodiment for measuring a hit rate and/or inspiratory airflow, e.g. based on an acoustic and/or motion signal caused by the subject's breathing, e.g. in response to electrical nerve stimulation. The system 100 of Fig. 2 shows that both the acoustic sensing unit 400 and/or the motion sensing unit 600 and the control unit 500 are parts of the external device 300. In this case, the acoustic sensing unit 400 may preferably be a microphone enclosed in the housing of the external device.

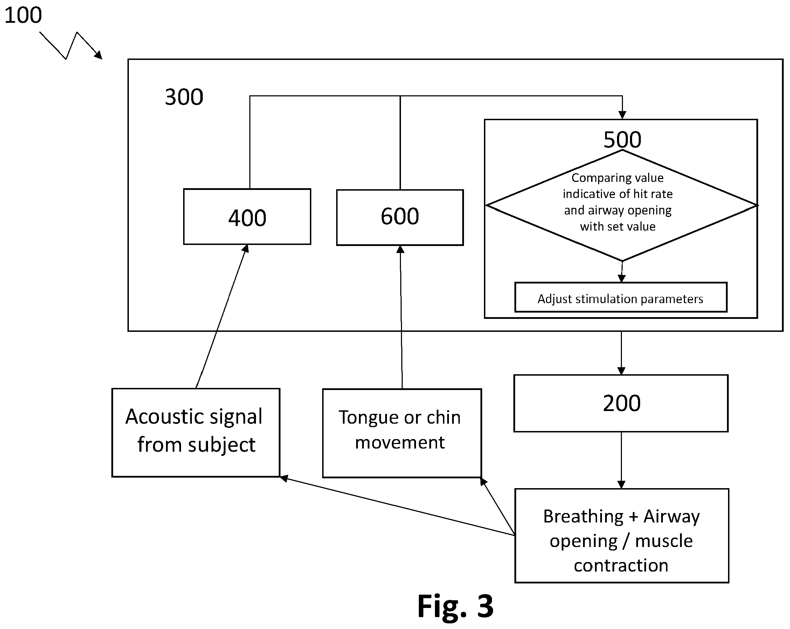

Fig. 3 depicts a flow diagram of a third embodiment of a system 100 for measuring a hit rate and/or inspiratory airflow, e.g. based on an acoustic and/or motion signal caused by the subject's breathing, e.g. in response to electrical nerve stimulation The system 100 of Fig. 3 is further specified in that a motion sensing unit 600 for detecting muscle contraction is implemented in addition to the acoustic sensing unit 400.

In order to determine a movement (i.e. a contraction) of one or more muscles related to the subject's breathing pattern, e.g. in response to the electric field applied by the implant unit 200, the system 100 comprises a motion sensing unit 600, which comprises at least one inertial measurement unit and/or at least one strain gauge. The inertial measurement unit may comprise at least one accelerometer and/or at least one gyroscope and/or at least one magnetometer. For example, the degree of protrusion of the subject's tongue can for example be determined by detecting movement of the muscle associated with the tongue or by detecting movement of the subject's chin. Automatic adjustment may comprise the control unit 500 receives a value indicative of inspiratory airflow from either the motion sensing unit 600 and/or the acoustic sensing unit 400, and thereupon adjusts at least one stimulation parameter, either after a set time delay or more or less instantly (i.e. in real-time).

The invention is not limited to one of the embodiments described herein but may be modified in numerous other ways.

All features disclosed by the claims, the specification and the figures, as well as all advantages, including constructive particulars, spatial arrangements and methodological steps, can be essential to the invention either on their own or by various combinations with each other.

### List of reference numerals

- 100: System
- 200: Implant unit
- 300: External device
- 400: Acoustic sensing unit
- 401: Inertial measurement unit
- 402: Strain gauge
- 500: Control unit
- 600: Motion sensing unit

## Claims

1. A method for sensing a subject's breathing, the method comprising:
- measuring an acoustic signal and a motion signal caused by the subject's breathing during a session of electrical nerve stimulation of the subject's hypoglossal nerve or ansa cervicalis, wherein the motion signal comprises a movement or contraction of one or more muscles related to the subject's airway;
- assigning to the measured acoustic signal and to the measured motion signal a value that is indicative of a hit rate and of an inspiratory airflow, wherein the hit rate is a quantification of a level of stimulation synchronization with the subject's inhalation phases based on the subject's breathing pattern,
wherein the movement of the one or more muscles related to the subject's airway is determined using at least one motion sensing unit, wherein the motion sensing unit comprises at least one inertial measurement unit,
wherein the inertial measurement unit comprises at least one magnetometer.

2. The method according to claim 1, wherein the method further comprises:
- generating a modulation signal, the modulation signal comprising an electrical stimulation pattern;
- applying the modulation signal to at least one pair of electrodes associated with an implant unit implanted inside the subject's body;
- adjusting at least one parameter of the electrical stimulation pattern in response to the value indicative of the measured hit rate and inspiratory airflow.

3. The method according to claims 1 or 2, wherein the acoustic signal comprises breathing sounds of the subject.

4. The method according to any of the preceding claims, wherein the acoustic signal is measured using at least one acoustic sensing unit, wherein the acoustic sensing unit may in particular comprise at least one microphone.

5. The method according to any one of claims 2 to 4, wherein the movement or contraction of the muscles related to the subject's airway comprises an airway muscle contraction, a change of position of the subject's tongue and/or a change of the tongue's spatial orientation within the subject.

6. The method according to claim 6, wherein the inertial measurement unit comprises at least one accelerometer and/or at least one gyroscope.

7. The method according to any of claims 2 to 6, wherein at least one parameter comprise a pulse train amplitude, a pulse train length, a single pulse frequency, a single pulse duration, a hold duration, a pulse train interval, a duty cycle, a delay time, a ramp duration, a step-down amplitude, a ramp at train onset duration, and/or a confirmatory pulse/train amplitude.

8. A system for implementing the method according to any of the preceding claims, the system comprising:
- an implant unit configured for implantation in the vicinity of the any muscle associated with the subject's airway opening,
wherein the implant unit comprises a pair of electrodes;
- an external device configured for communication with the implant unit;
- at least one acoustic sensing unit configured for measuring an acoustic signal;
- at least one motion sensing unit configured for measuring a movement of one or more muscles related to the subject's airway, the at least one motion sensing unit comprising at least one inertial measurement unit, wherein the at least one inertial measurement unit comprises a magnetometer; and
- a control unit configured to assign to the measured acoustic signal and to the measured motion signal a value that is indicative of a hit rate and of an inspiratory airflow,
and wherein the control unit is further configured to transmit stimulation input to the implant unit,
wherein the hit rate is a quantification of a level of stimulation synchronization with the subject's inhalation phases based on the subject's breathing pattern.

9. The system of claim 8, wherein the at least one acoustic sensing unit is part of the external device and wherein the at least one motion sensing unit is part of the external device or of the implant device.

## Patentansprüche

1. Verfahren zum Erfassen der Atmung eines Probanden, wobei das Verfahren Folgendes umfasst:
- Messen eines akustischen Signals und eines Bewegungssignals, die durch Atmen des Probanden während einer Sitzung zur elektrischen Nervenstimulation des Zungenmuskelnervs oder der Ansa cervicalis des Probanden verursacht werden, wobei das Bewegungssignal eine Bewegung oder Kontraktion eines oder mehrerer Muskeln in Bezug auf den Atemweg des Probanden umfasst;
- Zuweisen eines Werts, der indikativ für eine Trefferrate und einen inspiratorischen Luftstrom ist, zu dem gemessenen akustischen Signal und zu dem gemessenen Bewegungssignal, wobei die Trefferrate eine Quantifizierung eines Pegels einer Stimulationssynchronisation mit den Inhalationsphasen des Probanden basierend auf dem Atmungsmuster des Probanden ist,
wobei die Bewegung des einen oder der mehreren Muskeln in Bezug auf den Atemweg des Probanden unter Verwendung mindestens einer Bewegungserfassungseinheit bestimmt wird, wobei die Bewegungserfassungseinheit mindestens eine Trägheitsmesseinheit umfasst,
wobei die Trägheitsmesseinheit mindestens ein Magnetometer umfasst.

2. Verfahren nach Anspruch 1, wobei das Verfahren ferner Folgendes umfasst:
- Erzeugen eines Modulationssignals, wobei das Modulationssignal ein elektrisches Stimulationsmuster umfasst;
- Anlegen des Modulationssignals an mindestens ein Paar von Elektroden, die mit einer Implantateinheit assoziiert sind, die im Körper des Probanden implantiert ist;
- Anpassen mindestens eines Parameters des elektrischen Stimulationsmusters als Reaktion auf den Wert, der indikativ für die gemessene Trefferrate und den inspiratorischen Luftstrom ist.

3. Verfahren nach Anspruch 1 oder 2, wobei das akustische Signal Atemgeräusche des Probanden umfasst.

4. Verfahren nach einem der vorhergehenden Ansprüche, wobei das akustische Signal unter Verwendung mindestens einer akustischen Erfassungseinheit gemessen wird, wobei die akustische Erfassungseinheit insbesondere mindestens ein Mikrofon umfassen kann.

5. Verfahren nach einem der Ansprüche 2 bis 4, wobei die Bewegung oder Kontraktion der Muskeln in Bezug auf den Atemweg des Probanden eine Atemwegsmuskelkontraktion, eine Änderung der Position der Zunge des Probanden und/oder eine Änderung der räumlichen Ausrichtung der Zunge innerhalb des Probanden umfasst.

6. Verfahren nach Anspruch 6, wobei die Trägheitsmesseinheit mindestens einen Beschleunigungsmesser und/oder mindestens ein Gyroskop umfasst.

7. Verfahren nach einem der Ansprüche 2 bis 6, wobei mindestens ein Parameter eine Pulszugamplitude, eine Pulszuglänge, eine einzelne Pulsfrequenz, eine einzelne Pulsdauer, eine Haltedauer, ein Pulszugintervall, ein Tastverhältnis, eine Verzögerungszeit, eine Rampendauer, eine Abwärtsamplitude, eine Rampendauer bei Zugeinsatz und/oder eine bestätigende Puls-/Zugamplitude umfasst.

8. System zum Implementieren des Verfahrens nach einem der vorhergehenden Ansprüche, wobei das System Folgendes umfasst:
- eine Implantateinheit, die zur Implantation in der Nähe des beliebigen Muskels ausgelegt ist, der mit der Atemwegsöffnung des Probanden assoziiert ist,
wobei die Implantateinheit ein Paar von Elektroden umfasst;
- eine externe Vorrichtung, die für Kommunikation mit der Implantateinheit ausgelegt ist;
- mindestens eine akustische Erfassungseinheit, die zum Messen eines akustischen Signals ausgelegt ist;
- mindestens eine Bewegungserfassungseinheit, die zum Messen einer Bewegung einer oder mehrerer Muskeln in Bezug auf den Atemweg des Probanden ausgelegt ist, wobei die mindestens eine Bewegungserfassungseinheit mindestens eine Trägheitsmesseinheit umfasst, wobei die mindestens eine Trägheitsmesseinheit ein Magnetometer umfasst; und
- eine Steuereinheit, die dazu ausgelegt ist, dem gemessenen akustischen Signal und dem gemessenen Bewegungssignal einen Wert zuzuweisen, der indikativ für eine Trefferrate und einen inspiratorischen Luftstrom ist,
und wobei die Steuereinheit ferner dazu ausgelegt ist, eine Stimulationseingabe an die Implantateinheit zu übertragen,
wobei die Trefferrate eine Quantifizierung eines Niveaus einer Stimulationssynchronisation mit den Inhalationsphasen des Probanden basierend auf dem Atmungsmuster des Probanden ist.

9. System nach Anspruch 8, wobei die mindestens eine akustische Erfassungseinheit Teil der externen Vorrichtung ist und wobei die mindestens eine Bewegungserfassungseinheit Teil der externen Vorrichtung oder der Implantatvorrichtung ist.

## Revendications

1. Procédé de détection de la respiration d'un sujet, le procédé comportant les étapes consistant à :
- mesurer un signal acoustique et un signal de mouvement causés par la respiration du sujet pendant une séance de neurostimulation électrique du nerf hypoglosse ou de l'anse cervicale du sujet, le signal de mouvement comportant un mouvement ou une contraction d'un ou de plusieurs muscles associés aux voies aériennes du sujet ;
- affecter au signal acoustique mesuré et au signal de mouvement mesuré une valeur qui est indicative d'un taux de concordance et d'un débit d'air inspiratoire, le taux de concordance étant une quantification d'un niveau de synchronisation de la stimulation avec les phases d'inhalation du sujet sur la base du profil de respiration du sujet,
le mouvement du ou des muscles associés aux voies aériennes du sujet étant déterminé à l'aide d'au moins une unité de détection de mouvement, l'unité de détection de mouvement comportant au moins une unité de mesure inertielle,
l'unité de mesure inertielle comportant au moins un magnétomètre.

2. Procédé selon la revendication 1, le procédé comportant en outre les étapes consistant à :
- générer un signal de modulation, le signal de modulation comportant un profil de stimulation électrique ;
- appliquer le signal de modulation à au moins une paire d'électrodes associée à une unité d'implant implantée à l'intérieur du corps du sujet ;
- ajuster au moins un paramètre du profil de stimulation électrique en réponse à la valeur indicative du taux de concordance et du débit d'air inspiratoire mesurés.

3. Procédé selon les revendications 1 ou 2, le signal acoustique comportant des sons de respiration du sujet.

4. Procédé selon l'une quelconque des revendications précédentes, le signal acoustique étant mesuré à l'aide d'au moins une unité de détection acoustique, l'unité de détection acoustique pouvant en particulier comporter au moins un microphone.

5. Procédé selon l'une quelconque des revendications 2 à 4, le mouvement ou la contraction des muscles associés aux voies aériennes du sujet comportant une contraction de muscle des voies aériennes, un changement de position de la langue du sujet et/ou un changement de l'orientation spatiale de la langue chez le sujet.

6. Procédé selon la revendication 6, l'unité de mesure inertielle comportant au moins un accéléromètre et/ou au moins un gyroscope.

7. Procédé selon l'une quelconque des revendications 2 à 6, au moins un paramètre comportant une amplitude de train d'impulsions, une longueur de train d'impulsions, une fréquence d'impulsion individuelle, une durée d'impulsion individuelle, une durée de maintien, un intervalle de trains d'impulsions, un cycle de marche, un temps de retard, une durée de rampe, une amplitude d'abaissement, une durée de rampe au début d'un train, et/ou une amplitude d'impulsion/de train de confirmation.

8. Système de mise en œuvre du procédé selon l'une quelconque des revendications précédentes, le système comportant :
- une unité d'implant configurée en vue d'une implantation au voisinage du muscle quelconque considéré associé à l'ouverture des voies aériennes du sujet,
l'unité d'implant comportant une paire d'électrodes ;
- un dispositif externe configuré en vue d'une communication avec l'unité d'implant ;
- au moins une unité de détection acoustique configurée pour mesurer un signal acoustique ;
- au moins une unité de détection de mouvement configurée pour mesurer un mouvement d'un ou de plusieurs muscles associés aux voies aériennes du sujet, l'unité ou les unités de détection de mouvement comportant au moins une unité de mesure inertielle, l'unité ou les unités de mesure inertielle comportant un magnétomètre ; et
- une unité de commande configurée pour affecter au signal acoustique mesuré et au signal de mouvement mesuré une valeur qui est indicative d'un taux de concordance et d'un débit d'air inspiratoire,
et l'unité de commande étant en outre configurée pour transmettre une entrée de stimulation à l'unité d'implant,
le taux de concordance étant une quantification d'un niveau de synchronisation de la stimulation avec les phases d'inhalation du sujet sur la base du profil de respiration du sujet.

9. Système selon la revendication 8, l'unité ou les unités de détection acoustique faisant partie du dispositif externe et l'unité ou les unités de détection de mouvement faisant partie du dispositif externe ou du dispositif d'implant.
